(19) Europäisches Patentamt
European Patent Office
Office européen des brevets

(11) EP 0 643 683 B1

(12) EUROPEAN PATENT SPECIFICATION

(45) Date of publication and mention of the grant of the patent: **20.08.1997 Bulletin 1997/34**

(21) Application number: **93912709.8**

(22) Date of filing: **14.05.1993**

(51) Int. Cl.[6]: **C07C 45/50**, C07C 45/85, C07C 45/78, C07C 45/80

(86) International application number: **PCT/EP93/01209**

(87) International publication number: **WO 93/24436 (09.12.1993 Gazette 1993/29)**

# (54) METHOD AND APPARATUS FOR THE RECOVERY OF METAL CATALYSTS

VERFAHREN UND VORRICHTUNG ZUR WIEDERGEWINNUNG VON METALLKATALYSATOREN

PROCEDE ET APPAREIL DE RECUPERATION DES CATALYSEURS METALLIQUES

(84) Designated Contracting States: **FR NL**

(30) Priority: **04.06.1992 GB 9211880**

(43) Date of publication of application: **22.03.1995 Bulletin 1995/12**

(73) Proprietor: **EXXON CHEMICAL PATENTS INC.**
**Linden, New Jersey 07036-0710 (US)**

(72) Inventors:
- **HANIN, Jean, Alexandre, Andre**
  **B-1330 Rixensart (BE)**
- **VAN DRIESSCHE, Eddy**
  **B-9900 Eekloo (BE)**

(74) Representative: **Bawden, Peter Charles et al**
**EXXON CHEMICAL LIMITED**
**European Patents and Licences**
**Exxon Chemical Technology Centre**
**P.O. Box 1**
**Abingdon Oxfordshire OX13 6BB (GB)**

(56) References cited:

| | |
|---|---|
| EP-A- 0 183 546 | FR-A- 2 235 087 |
| GB-A- 2 055 371 | US-A- 3 725 534 |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.14.12/3.4

## Description

This invention relates to the oxo process and, in particular, to the catalyst cycle of the oxo process. That is, it relates to a method for removing metal-containing catalyst residues, for example, cobalt-containing residues, from crude oxo products and recovering therefrom catalyst residues in a form suitable for recycle to the oxo reaction.

Oxo reactions (also referred to as hydroformylation reactions) involve the preparation of oxygenated organic compounds by the reaction of a carbon monoxide and hydrogen mixture ("syn gas") with carbon compounds containing olefinic unsaturation. The reaction is performed in the presence of a carbonylation catalyst and results in the formation of a compound, for example, an aldehyde, which has one more carbon atom in its molecular structure than the starting olefinic feedstock. By way of example, higher alcohols may be produced in the oxo process by hydroformylation of commercial $C_6$-$C_{12}$ olefin fractions to an aldehyde-containing oxonation product, which on hydrogenation yields $C_7$-$C_{13}$ alcohols. The crude product of the hydroformylation reaction will contain catalyst, aldehydes, alcohols, unreacted feedstock, syn gas and by-products.

Before further processing of the crude product is possible, it is necessary to remove the catalyst therefrom. One conventional method of removing cobalt values (that is, cobalt in any one of its possible oxidation states) from such a crude product is to treat the product with an alkali or acid wash technique; however, this uses expensive raw materials and, moreover, it is difficult to remove essentially all traces of cobalt from the water wash streams before those may be discharged into water courses. Another such conventional method involves the oxidation of the cobalt catalytic species followed by extraction as a salt in aqueous solution.

US Patent No. 3725534 proposes a method for recovery of cobalt values from crude oxo product in which, after separation from unreacted gases, the oxo product containing substantially all the cobalt values in the form of dissolved cobalt compounds is mixed with an acidic solution, the cobalt materials being converted to water-soluble salts of the acid. After it has been combined with a promotor, for example, an alkali or alkaline earth metal salt of a weak acid, the separated aqueous phase is introduced into a preformer containing a preforming catalyst, for example, a noble metal catalyst. The amount of the promotor is so selected that substantially complete carbonylation of the cobaltous ions present is achieved. The effluent is then preferably treated with a strong mineral acid to convert complex cobalt carbonyl compounds to volatile hydrocobalt carbonyl, which is stripped out of the acidified effluent by an inert gas, absorbed in an organic solvent and fed to the oxonation reactor. Although it is stated that the complex carbonyl compounds may be recycled direct to the oxo reactor without prior acid treatment, that is for practical reasons undesirable as they are inactive as oxonation catalysts and they introduce undesirable impurities, which could interfere with the oxo reaction, possibly leading to plugging of the reactor.

It is known to remove volatile cobalt compounds from crude hydroformylation reaction products by stripping them out with a countercurrent of syn gas. The cobalt contained in the crude product is in the form of complex compounds of cobalt with carbon monoxide, known as cobalt carbonyls, which are either formed during the principal reaction from the cobalt metal or compound introduced into the hydroformylation reactor, or which are introduced into the reactor direct in the form of carbonyls. Those cobalt carbonyls are readily soluble in organic liquids (such as the organic products of the oxo process) when in the form, for example, of the highly volatile hydrocobalt carbonyl ($HCo[CO]_4$), or in the form of dicobalt octacarbonyl $Co_2(CO)_8$, which has a low volatility; however, the exact species of the cobalt complex in the crude product depends to a large extent upon the temperature and pressure conditions obtaining. For example, in the presence of hydrogen, dicobaltoctacarbonyl (which is solid at room temperature) reaches an equilibrium with hydrocobalt carbonyl (which is highly volatile at room temperature) the concentration of dicobalt octacarbonyl being low at high temperatures and high hydrogen partial pressures. At high temperatures and low carbon monoxide partial pressures, however, both of those carbonyls decompose with precipitation of cobalt or cobalt oxide. Hitherto known stripping techniques have in general had to take account of the equilibria established in the cobalt/carbon monoxide complex system, and accordingly gas stripping techniques using specified conditions have been necessary.

In U.S. Patent No. 4625067, there is disclosed a method of removing cobalt values from the crude product of a cobalt-catalysed hydroformylation reaction by contacting the crude product with a stream of stripping gas to entrain volatile cobalt compounds, said contacting being performed in the presence of water or aqueous acid to dissolve those cobalt values not entrained in the gas under the conditions of temperature and pressure employed for said contacting, and the aqueous phase subsequently being separated from the organic hydroformylation reaction product. The separated cobalt-containing aqueous phase is concentrated in a flash unit, and the concentrate recycled to the oxo reactor. Optionally, however, the concentrate is, instead of being recycled to the reactor, passed to a preformer unit, where it is mixed with fresh cobalt, and volatile cobalt carbonyls are removed by means of a flow of gas, preferably syn gas, injected into the unit. Those cobalt carbonyls are carried by the gas to the oxo reactor.

The present invention provides a method of recovery of cobalt values from a cobalt-containing aqueous phase, comprising contacting the aqueous phase, in the presence of syn gas, with a solid catalyst that is suitable for catalysing carbonylation of cobalt ions and with an organic solvent in which hydrocobaltcarbonyl and dicobalt octacarbonyl are soluble, the said solvent being substantially immiscible with water and being present in an amount of not less than 20% by volume, based on the combined volume of the organic solvent and the aqueous phase to be contacted with the solid

catalyst, and recovering from the aqueous phase and organic liquid phase so obtained volatile cobalt carbonyl compounds for use as hydroformylation catalysts.

(For the sake of clarity, organic phases that are in liquid form are referred to throughout the specification as "organic liquid phases". It will be appreciated that the aqueous phases referred to herein are all in the liquid phase.)

Preferably, the aqueous phase and the organic liquid phase are treated in a stripping step in which the said phases are contacted with a stripping gas, volatile cobalt compounds being entrained in the stripping gas.

Advantageously, the aqueous phase and the organic liquid phase are combined with fresh oxo product from an oxo reactor before they are contacted with the stripping gas.

The invention further provides a method for recovery of cobalt values from the crude product of a cobalt-catalysed oxo reaction, the crude oxo product containing cobalt compounds in addition to an organic oxo product, the method comprising

treating the crude oxo product to remove volatile cobalt compounds;
contacting the crude oxo product with an aqueous phase of pH not greater than 7 to form an aqueous phase containing cobalt values; and
contacting the aqueous phase so obtained, in the presence of syn gas, with a solid catalyst that is suitable for catalysing carbonylation of cobalt ions and with an organic solvent for hydrocobalt carbonyl and dicobaltoctacarbonyl, the said solvent being substantially immiscible with water, to obtain cobalt-containing aqueous and organic liquid phases and a cobalt-containing gas phase.

By contacting a cobalt-containing aqueous phase in the presence of syn gas, with a catalyst for carbonylation of cobalt ions and with an organic solvent for hydrocobalt carbonyl and dicobaltoctacarbonyl, it is possible for cobalt to be recovered for recycling to the oxo reactor within a substantially closed cycle, that is, with little or no loss of cobalt from the system and with the generation of reduced amounts of waste water in comparison with, for example, the process of US Patent Specification No. 3725534.

It will be appreciated that the defined cobalt recovery method may be employed as a process step in an oxo process comprising hydroformylating an olefinic feedstock with syn gas in the presence of a cobalt-containing catalyst to form a product mixture containing higher aldehyde, alcohol, unreacted feed and secondary products. The decobalting method is employed prior to further treatment of the oxo product mixture.

Preferably, at least one of the cobalt-containing syn gas and the cobalt-containing aqueous and organic liquid phases obtained is treated to remove volatile cobalt compounds therefrom. In one particularly advantageous form of the invention the crude oxo product is treated in a stripping step in which it is contacted with a stream of stripping gas in the presence of an aqueous phase of pH not greater than 7, to entrain volatile cobalt compounds in said stripping gas and to form an aqueous phase containing cobalt values. The aqueous phase so obtained is then contacted in a further step (hereinafter referred to as the "preforming step") with a solid catalyst in the presence of an organic liquid phase and syn gas, as will be described in more detail below. Advantageously, after the preforming step, the cobalt-containing aqueous and organic liquid phases and the cobalt-containing syn gas are treated to remove volatile cobalt compounds therefrom, the treatment preferably comprising recycling one or more of that gas and those phases to the said stripping step. The cobalt-containing aqueous and organic liquid phases and the cobalt-containing syn gas obtained in the preforming step are advantageously combined with fresh crude hydroformylation product and fed therewith to the stripping step. Cobalt values that have been converted to volatile cobalt carbonyls in the preforming step may then be entrained in the stripping gas and returned to the oxo reactor while unconverted cobalt values are once again removed with the aqueous phase for recycling to the preforming step.

As the stripping gas in the stripping step there is preferably used syn gas, the particular proportions of the carbon monoxide and hydrogen in the syn gas being adjusted to suit the reaction system. The stripping gas may, though, be any gas that does not react in undesired manner with the cobalt compounds to be entrained therein, for example, nitrogen or a light saturated hydrocarbon such as propane or butane.

It is preferred in accordance with the invention that the volume ratio of stripping gas to crude product at the applied conditions is from 20:1 to 250:1, more preferably 50:1 to 125:1. Higher ratios may be used, with good effect, but high ratios may be detrimental to the economic basis of the process. The limiting lower value of the ratio, it will be appreciated, will be reached when there is insufficient stripping gas flow to achieve the desired degree of cobalt volatiles removal. Similarly any flow rate of stripping gas may be used, provided that it is sufficient to give the desired entrainment of cobalt volatiles. The liquid (organic and aqueous) phases are preferably well dispersed to give good contact between the liquids. It is preferred, too, that the stripper unit in which the stripping step is performed should include inert solid surfaces, for example, trays, to facilitate contact between the liquid and gas phases.

The stripping step is performed in the presence of water or an aqueous organic acid, and cobalt salts formed are taken up in the aqueous phase. It is therefore unnecessary to maintain a tight control on the temperature and pressure conditions in order to adjust the dicobaltoctacarbonyl/hydrocobalt carbonyl equilibrium, so as to prevent the cobalt carbonyl decomposition that leads to deposition of solids.

In the case where water (rather than aqueous acid) is present in the stripping step, then cobalt may be transformed into $Co[Co(CO)_4]_2$ but, because of the presence of formic acid, which is formed in the oxo reaction and present in the oxo product, the $Co[Co(CO)_4]_2$ is transformed into cobalt formate or hydrocobalt carbonyl. Any suitable organic acid may be used, for example, acetic acid or formic acid. It is especially preferred that the aqueous phase should comprise dilute formic acid, for example, at concentrations of from 1 to 5, especially from 1 to 2 wt. %. Preferably, the water or aqueous acid is present in an amount of from 5 to 15 wt. % based on the amount of crude product.

As mentioned above, it is preferred to employ an acidic aqueous phase in the stripping step and reference is made to the following reaction equations by way of explanation:

$$\underset{(a)}{2HCo(CO)_4} \rightleftarrows \underset{(b)}{Co_2(CO)_8} + H_2 \quad (1)$$

$$3Co_2(CO)_8 \longrightarrow \underset{(c)}{2Co[Co(CO)_4]_2} + 8CO \quad (2)$$

$$Co[Co(CO)_4]_2 + 2HCOOH \underset{}{\overset{\text{Aq. acid}}{\rightleftarrows}} \underset{(d)}{2Co(CO)_4^-} + \underset{(e)}{Co^{2+}} + 2HCOOH \quad (3)$$

$$Co(CO)_4^- + H^+ \overset{\text{Aq. acid}}{\rightleftarrows} HCo(CO)_4 + OH^- \quad (4)$$

Equilibrium (1) exists in the organic liquid phase, and in use of the method (a) is removed by the stripping gas. Some (b) is also present and under the temperature and pressure conditions employed reacts according to reaction (2) to give (c) which is preferentially extracted into the aqueous phase. In a neutral environment (c) remains in the aqueous phase. In an aqueous acidic environment, however (described here with reference to formic acid), equilibrium schemes (3) and (4) apply. Thus the acid proton combines with (d) to form the more volatile (a) in the aqueous phase, and (a) is removed by means of the stripping gas directly from the aqueous phase or following its extraction into the organic liquid phase. The acid anion (formate) functions to solubilise the $Co^{2+}$ cation (e) in the aqueous phase as cobalt formate solution. Thus the concentration of hydrocobalt carbonyl present in the aqueous phase (as hydrocobalt carbonyl (a) per se or as its anion (d)) is reduced in an advantageous manner when the aqueous phase is acidic; the amount of volatile cobalt remaining in the aqueous phase (and in the organic liquid phase) is decreased to minimal amounts. It is particularly preferred to use aqueous formic acid as the aqueous phase required to be present in the stripping step, as formic acid is one of the organic compounds produced in the hydroformylation reaction and its associated secondary reactions to which this invention primarily relates.

The stripping step may be carried out at relatively low pressures, and preferably the method is conducted at pressures lower than the pressure at which, under the temperature conditions employed, the cobalt compounds present in the crude oxo reaction product will decompose. More preferably the pressure is below 20 atmospheres ($2 \times 10^6$ Pa), most preferably below 10 atmospheres ($10^6$ Pa), especially for example a pressure below 7 atmospheres such as from 1 to 5 atmospheres ($10^5$ to $5 \times 10^5$ Pa). The temperature employed is preferably no more than 120°C, more preferably from 90 to 100°C. The optimum temperature will depend on the pressure employed.

For example, stripping may be performed at about 95°C and about 1 atmosphere gauge ($10^5$ Pa gauge) pressure, using syn gas as the stripping gas, in a volume ratio of about 100:1 at the stripping conditions compared with the total liquid flow.

Volatile cobalt compounds entrained in the stripping gas are advantageously utilised in the hydroformylation reaction. Preferably, after it has been contacted with the crude product, the stripping gas is contacted with olefin to be fed to the oxo reactor, the stripping gas advantageously being passed in countercurrent to the olefinic feed. The volatile cobalt compounds entrained in the stripping gas dissolve in the olefinic feed and are thus returned to the oxo reaction zone. The treated stripping gas may then either be purged or recycled for further contact with the crude product. It will be appreciated therefore that syn gas is the preferred stripping gas because its subsequent recycle contact with the olefinic feedstock will not cause the introduction of undesirable materials into the oxo reactor. Under the conditions employed, substantially no water will be carried over by the stripping gas (syn gas) and introduced into the olefinic feed. If necessary the cobalt-containing stripping gas may be cooled prior to contact with the olefinic feed, and condensed

water refluxed into the stripper.

Introduction of, for example, 5 to 15 wt. % water or dilute organic acid, preferably formic acid, into the crude oxo product results in some 66 to 90% of the cobalt being removed in the stripping gas with substantially all the balance of 34 to 10% of cobalt being separated from the oxo product in the stripper as cobalt salt, preferably formate, in aqueous solution, which aqueous solution may be treated in accordance with the invention and recycled to the stripping step. Residual cobalt in the treated oxo product may be removed by any suitable method, for example, by means of a wash tower as described in US Patent No. 4625067, the cobalt material being recycled to the stripping step in the aqueous effluent from the wash tower.

After contacting the crude oxo product with the stripping gas and the water or aqueous acid, the aqueous phase is separated from the treated oxo product.

Preferably, the aqueous phase so obtained, which may especially advantageously have been obtained using the stripping step described above, is concentrated before being passed to the reactor (preformer) containing the solid catalyst. The aqueous concentrate advantageously contains the maximum possible amount of solubilised cobalt for the particular operating temperature. If formic acid has been employed in the stripping step, the aqueous phase is preferably concentrated to give a cobalt concentration of from 0.5 to 2 wt %, based on the total concentrate, expressed as elementary cobalt. Advantageously, the concentration of free formic acid in the concentrate is from 2 to 8 wt % based on the total concentrate.

The concentrate may be produced by flashing the aqueous phase in an evaporator or by using any other convenient means, for example, a membrane, to yield the concentrate, for example, a cobalt formate/formic acid aqueous solution, and an aqueous effluent. It is especially advantageous for the aqueous effluent to be recycled to the stripping step to form a part of the aqueous phase with which the oxo product is contacted, the effluent preferably being passed to the stripping step via a wash tower as described in US Patent No. 4625067. Preferably, the water stream fed to the wash tower is exclusively aqueous effluent from the evaporator. If desired, fresh water make-up, for example, in an amount of 1 to 3% by volume, based on the oxo product, may be added to the aqueous effluent from the evaporator that is fed to the wash tower. In that manner, water from the evaporator may be retained within a substantially closed cycle, it being possible substantially to avoid the need to purge water from the evaporator. If it is necessary, for example, in order to remove undesired impurities, some aqueous effluent from the evaporator may be purged from the system.

As already mentioned it is preferred to use in the stripping step an aqueous formic acid solution, although other acids, for example acetic acid, may be used. For ease of explanation, it is assumed in the following explanation of the preforming step that formic acid is used in the stripping step.

The cobalt values contained in the aqueous phase obtained from the stripping step will be primarily in the form of cobalt formate, which, in the presence of a noble metal catalyst, for example, palladium, and syn gas is converted to $Co[Co(CO)_4]_2$ in accordance with the following reaction:

$$3Co(COOH)_2 + 3H_2 + 8CO \underset{}{\overset{Pd}{\rightleftarrows}} Co[Co(CO)_4]_2 + 6HCOOH \quad (5)$$

Hydrocobalt carbonyl is liberated from the $Co[Co(CO)_4]_2$ by the following reaction, formation of the hydrocobalt carbonyl being enhanced by the presence of free formic acid, which is produced as a by-product in the hydroformylation process as well as in the conversion of cobalt formate to $Co[Co(CO)_4]_2$, and will normally be present in the aqueous phase:

$$Co[Co(CO)_4]_2 + 2HCOOH \rightarrow 2HCo(CO)_4 + Co(COOH)_2 \quad (6)$$

Hydrocobalt carbonyl is relatively insoluble in water (maximum solubility at 20°C 3000 ppm, expressed as elemental cobalt). It is believed that as the concentration of the hydrocobalt carbonyl in the aqueous phase approaches a concentration corresponding to the said maximum solubility, the carbonyl separates from the aqueous phase and is extracted into the organic liquid phase. Some of the hydrocobalt carbonyl becomes entrained in the syn gas. Further hydrocobalt carbonyl may be transformed in accordance with equilibrium (1) above into dicobaltoctacarbonyl, which is extracted into the organic liquid phase. In the absence of an organic liquid phase, the dicobaltoctacarbonyl would precipitate onto the catalyst causing deactivation thereof. Some of the dicobaltoctacarbonyl formed is transformed in accordance with equilibrium (2) above to form $Co[Co(CO)_4]_2$, which is taken up in the aqueous phase. In consequence of equilibrium (2) it will be impossible for the preforming system to achieve full conversion of cobalt ions to cobalt carbonyl in a single extraction step.

As solid catalyst for promotion of the formation of cobalt carbonyl compounds there may be used, for example, any

metals of Group IB or Group VIII of the Periodic Table of the elements, for example, palladium, platinum or gold, the metals being present in elementary form or in an oxidation state greater than zero. The catalyst material may be provided on a solid support, for example, on carbon or alumina. Palladium and palladium compounds are preferred as the metal catalyst. Advantageously palladium supported on carbon (Pd/C) is used.

The organic solvent used in the preforming step may be any organic solvent that is substantially immiscible with water and that when introduced with oxo product to the stripping step does not interfere with the subsequent recovery of the products of the oxo process. The organic solvent may advantageously be olefin, decobalted crude oxo product (that is, the product obtained after treatment of crude oxo product to remove catalytic residues but without hydrogenation or distillation of the organic material) hydrogenated oxo product, oxo alcohols, light oxo fraction or heavy oxo fraction. As olefin there may be used any olefin or mixture of olefins that is liquid under the conditions obtaining in the preformer. Advantageously, the feed olefin to be used in the oxo reaction is used as the organic solvent. Preferably, however, decobalted crude oxo product or a distillation product derived from crude oxo product is used as the organic solvent. A hydrogenated oxo product, for example, a hydrogenated isodecyl oxo product containing a mixture of decanols may be used.

The ratio of organic liquid phase to aqueous phase in the preforming step may be from 0.2:1 to 5:1 V/V and may, for example, be 1:1 V/V. The amount of syn gas is preferably so selected that both hydrogen and carbon monoxide are present in a stoichiometric excess of at least 20% over the total cobalt in the preformer feed. The molar ratio of hydrogen to carbon monoxide may be from 0.5:1 to 2:1.

Advantageously, the aqueous phase obtained from the stripping step is combined with the organic solvent and the combined aqueous and organic liquid phases obtained are passed to a two-liquid-phase plug flow reactor having one or more fixed beds of Pd/C, to which syn gas is also fed. The reactor comprises means for effecting mixing of the two liquid phases and the gas phase so that the two liquid phases are well dispersed and the interface between the liquid phases and the gas phase is sufficiently large that the mass transfer of material from the liquid phases to the gas phase is not limited by the size of the said interface. The temperature in the reactor is preferably from 50 to 175°C, more preferably from 120 to 140°C. The pressure in the reactor may be from 100 to 300 atm g ($1 \times 10^7$ to $3 \times 10^7$ Pa g), and is preferably from 250 to 300 atm g ($2.5 \times 10^7$ to $3 \times 10^7$ Pa g).

Because the preferred preforming cycle described above allows essentially all the water to be in a closed loop, the waste water from the evaporator being recycled, preferably via a wash tower, to the stripping step, the need to dispose of waste water can be substantially avoided. Thus, the method of the invention enables environmentally clean cobalt recovery to be attained.

Further, because the invention provides an internal closed loop for any cobalt formate in the aqueous phase that remains unreacted in the preformer, it is not important for complete preforming conversion to be obtained. Cobalt values that have not been preformed (that is, converted to cobalt in an active form) are carried in the aqueous phase and recycled via the stripper and the evaporator to the preformer. It is therefore unnecessary to use a complex preformer that is capable of achieving complete conversion; for example, a two-liquid-phase concurrent plug flow preformer can be used. A countercurrent preformer with fixed catalyst bed could be used, if desired.

Also, the process may be operated without the addition of chemical reactants, other than the reactants for use in the oxo process, because formic acid, which promotes the conversion of $Co[Co(CO)_4]_2$ to hydrocobalt carbonyl, is produced in the oxo reaction. Thus, after an initial introduction of formic acid, it is possible to make the system substantially closed with regard to formic acid.

Using a simple preformer, for example, a two-liquid-phase concurrent plug flow preformer, an overall cobalt recovery of about 58%, based on the cobalt entering the preformer, may be achieved, with approximately 78% of cobalt values being converted in the preformer to volatile cobalt carbonyls and about 76% of that preformed cobalt being taken up by the stripping gas in the stripping step. Significantly more efficient recovery may be obtainable by using, for example, a countercurrent preformer with a fixed catalyst bed at high pressure.

Any cobalt loss may be made up by introducing cobalt, for example, in the form of cobalt soap, into the preformer with the organic phase or in the form of an organic cobalt salt, for example, cobalt formate, in aqueous solution.

The invention further provides an oxo apparatus comprising

an oxo reactor;
a stripping means arranged for receiving crude oxo product from the reactor and comprising means for effecting contact between the said oxo product and syn gas in the presence of water or aqueous acid and means for separating the aqueous phase from the oxo product and syn gas;
a preformer arranged for receiving the aqueous phase from the stripping means, the said preformer having means for containing a solid catalyst and having inlet means for the aqueous phase, for an organic liquid phase and for a gas, the preformer further comprising means for effecting contact between the liquid phases and the solid catalyst, and means for effecting contact between the aqueous phase and the organic liquid phase and the gas; and recycling means for recycling to the stripping means effluent from the preformer comprising one or more of the organic liquid phase, the aqueous phase and the gas.

Moreover, the invention provides the use of a two-liquid-phase catalytic preformer for improving the efficiency of recovery of cobalt values, the preformer containing a solid catalyst that is suitable for catalysing carbonylation of cobalt cations.

One method of cobalt recovery in accordance with the invention will now be described in greater detail by way of example only with reference to the accompanying drawings of which:

Figure 1 is a flow diagram of a hydroformylation reaction system;
Figure 2 is a bar graph illustrating catalytic cobalt conversion in a one-liquid-phase system; and
Figure 3 is a bar graph illustrating catalytic cobalt conversion in a two-liquid-phase system.
Fig. 4A is a bar graph showing cobalt distribution after cooling and reactor discharge in a one-liquid-phase system; and
Fig. 4B is a bar graph showing cobalt distribution after cooling and reactor discharge in a two-liquid-phase system.

Referring to Figure 1, feed olefin and syn gas are delivered in a desired ratio via lines 1 and 3 respectively to an oxo-reactor 5, wherein hydroformylation is conducted under conventional conditions to yield a crude hydroformylation product containing aldehydes, alcohols, by-products and cobalt catalyst compounds. The crude product is carried via line 7 to a stripper unit 9. Also introduced into the stripper unit 9 are water from a wash tower 11 via line 13 and stripper gas via line 15. The stripper gas may be for example syn gas or nitrogen. In the stripper unit 9 the stripper gas contacts the crude hydroformylation product and entrains much of the volatile cobalt carbonyl compounds contained therein, carrying off the entrained material via line 17 to an absorber unit 19. In the absorber unit 19, the cobalt-containing stripper gas is run in countercurrent to a supply of feed olefin delivered from line 21 and, by virtue of its good solubility in the organic liquid phase, the volatile cobalt becomes absorbed in the olefin and carried via line 1 to the oxo reactor. The feed introduced into the reactor thus contains cobalt, effecting recycle of at least part of the cobalt used in the hydroformylation reaction. The stripping gas leaving the absorber 19 may be purged via line 23 or recycled to the stripper unit 9 via line 15. By virtue of the water introduced into the stripper unit via line 13, stripping is conducted in the presence of an aqueous phase, into which dissolves the water-soluble cobalt species not entrained by the stripping gas. Such aqueous phase is removed from the stripper 9 via line 25, into which air is optionally injected to oxidise any volatile cobalt carbonyls contained therein. The cobalt-containing aqueous phase is subjected to concentration in a flash unit 27, with water being removed overhead and recycled to the stripper unit 9 via the lines 29 and 35, wash tower 11 and line 13. Optionally, from 1 to 3% by volume based on the oxo product of fresh water make-up can be added from line 37. If any water is not recycled from flash unit 27 to the stripper unit 9, it may be purged from the system via line 41. Concentrated aqueous phase is removed from the flash unit via line 2.

The crude oxo product, having had substantially all its cobalt content removed in the stripper unit 9, is carried therefrom via line 33 to the wash tower 11, where water introduced via line 35 (which is recycled clean flash unit water from line 29 optionally including some make-up water introduced through line 37) is used in a conventional washing step for finally removing the remaining cobalt catalyst content of the oxo product. In the embodiment shown, such wash water is returned to the stripper via line 13. The thus decobalted oxo product is carried via line 39 to further process stages such as hydrogenation and distillation.

Because formic acid is produced during the oxo reaction, even if pure water is employed initially, the closed system described soon stabilises into a closed formic acid system, with the advantages described hereinbefore. Optionally, formic acid may be introduced into the system initially at any convenient point.

The concentrated aqueous phase leaving the flash unit 27 is delivered via line 2 to a cobalt catalyst preformer unit 4. The preformer unit 4 contains a noble metal catalyst and may be, for example, in the form of a tubular reactor having a fixed bed containing Pd/C. An organic phase, for example, feed olefin, oxo product, alcohol, light oxo fraction or heavy oxo fraction, is delivered to the preformer unit 4 via line 40. If necessary, a supply of cobalt make-up, for example, in the form of a solution of cobalt soap in a higher alcohol or of an organic cobalt salt in water is delivered to the preformer unit 4 via line 6. Syn gas is fed to the preformer unit 4 via line 10.

In the preformer unit 4 the gas phase, the organic liquid phase and the aqueous phase are preferably well mixed to give good contact between the liquids themselves, between the liquids and the gas phase, and between the liquid phases and the catalyst. Volatile cobalt carbonyls become entrained in the syn gas, and some cobalt dissolves in the organic liquid phase, leaving a proportion of the cobalt dissolved in the aqueous phase. Both liquid phases and the syn gas are delivered via the line 8 to the stripper 9 where cobalt values that have been converted to volatile cobalt carbonyls are removed by the stripping gas via line 17 and unconverted cobalt values are taken up in the aqueous phase and thus recycled to preformer unit 4 via flash unit 27.

If desired, instead of feeding both liquid phases and the gas phase to the stripper unit 9, the organic liquid phase may be separated from the aqueous phase and the gas phase and fed direct to the oxo reactor, only the aqueous phase and the gas phase being fed to the stripper unit 9; a separator in line 17 and a further line for feeding the organic liquid phase to the oxo reactor (neither of which is shown in Fig. 1) are then provided.

The following Examples illustrate the invention:

Example I

An aqueous phase containing I.02 wt % cobalt and isodecanol was continuously fed to a preformer unit. Syn gas, consisting of hydrogen and carbon monoxide in a molar ratio of I.3:1, was also continuously introduced into the preformer at a flow rate of 1.1 l/h, measured under the preformer conditions. The preformer unit contained three continuously stirred tank reactors arranged in series, each having a catalyst basket containing I30 ml of Pd/C. Such an arrangement permits a level of conversion to be obtained on a laboratory scale that is similar to that obtained on a production scale using a single tubular plug flow reactor. The ratio of aqueous phase to alcohol phase was 0.88:1 V/V. The flow of the combined aqueous and organic liquid phases was such that the volume of liquid per hour/total volume of catalyst was 2.4 $h^{-1}$. The temperature in the preformer was I20°C and the pressure was 300 bar g (300 x $10^5$Pa gauge).

Effluent comprising aqueous phase, alcohol phase and syn gas was withdrawn from the preformer unit and delivered to a stripper tower. The cobalt content of the aqueous feed to the preformer unit and of the effluent from the preformer unit was ascertained. The pressure within the stripper tower was 2.3 bar g (2.3 x $10^5$ Pa gauge) and the flow rate of the stripping gas was maintained at II6 l/h measured at the stripping conditions. The liquid flow rate (combined aqueous and alcohol phases) was 0.9I6 1/h. The ratio of stripping gas to liquid phase was I27:1 V/V at the applied conditions and the ratio of aqueous to alcohol phase was 0.88:1 V/V.

The alcohol phase and aqueous phase leaving the bottom of the stripper were separated and the cobalt content of the aqueous phase ascertained. The aqueous phase was recycled to the preformer, after being combined with fresh isodecanol. The preforming operation was run continuously for 58.I hours.

Table Ia illustrates the transfer of cobalt from the aqueous phase fed to the preformer to the organic phase.

Table Ia

| | |
|---|---|
| Total water phase fed to preformer | 25I6I g |
| Total alcohol phase fed to preformer | 23904 g (= 28628 $cm^3$) |
| Total cobalt in water feed | 257 g |
| Free water phase at outlet of preformer | 24563 g |
| Free alcohol phase at outlet of preformer | 24502 g |
| Average $Co^{2+}$ in water after preformer | 0.2407 wt% Co = 59.I2g Co (1) |
| Average preformed cobalt* in water after preformer | 0.3554 wt% Co = 87.30g Co (2) |
| Average preformed cobalt* in alcohol after preformer | 0.3620 wt% Co = 88.7 g Co (3) |

* "preformed cobalt" refers to the total combined amounts of hydrocobalt carbonyl, dicobaltoctacarbonyl and $Co(CO)^{4-}$ anion.
(I) + (2) + (3) = 9I.6% balance excluding cobalt in gas phase. (The balance of the cobalt (21.88g) is assumed to be in the syn gas.)

By adding together the quantities of cobalt in the alcohol phase and in the gas phase, together with the preformed cobalt in the aqueous phase (I98 g) it can be calculated that 77% of cobalt formate in the aqueous feed had been converted to cobalt carbonyls and that the proportion of preformed cobalt extracted by the alcohol phase was 45%.

Table Ib gives the overall cobalt balances of the combined preforming and stripping steps.

Table Ib

| | |
|---|---|
| Cobalt fed to preformer | 257 g Cobalt |
| Preformed cobalt leaving preformer | I98 g Cobalt |
| Cobalt recovered in stripping gas | I50 g Cobalt |
| Cobalt in bottom (aqueous) stream from stripper | I05 g Cobalt (2+) |
| Balance | 255 g = 99.2% |

Based on the amount of preformed cobalt (I98 g) present in the feed to the stripper tower, the stripper tower gives

a cobalt recovery (that is cobalt recovered in stripping gas) of 76%.

From the preforming conversion of 77% and the stripper recovery of 76%, an overall preforming/stripping conversion of 58.5% results.

Example 2

The effectiveness of palladium-catalysed conversion of cobalt values in a two-liquid-phase system was compared with conversion in a single-liquid-phase aqueous system.

(a) One-liquid-phase system

2.45 kg of cobalt-containing aqueous phase obtained from a stripping tower of the type described above with reference to Figure 1 was contacted in an autoclave with 180g catalyst (0.8% palladium on carbon) in the presence of syn gas ($H_2/CO$ = 1.3 : 1) at 120°C and a pressure of 300 atmospheres (about 3 x $10^7$ Pa). The aqueous phase had a pH of 2.7 due to the presence of 3% free formic acid in the water. The cobalt content of the aqueous phase was analysed at intervals over a period of 90 minutes, at which time the amount of cobalt in the aqueous phase was found to have dropped by about 30%. Figure 2 shows the $Co^{2+}$ and preformed cobalt of the samples taken. The upper cross-hatched areas represent the cobalt removed in sampling.

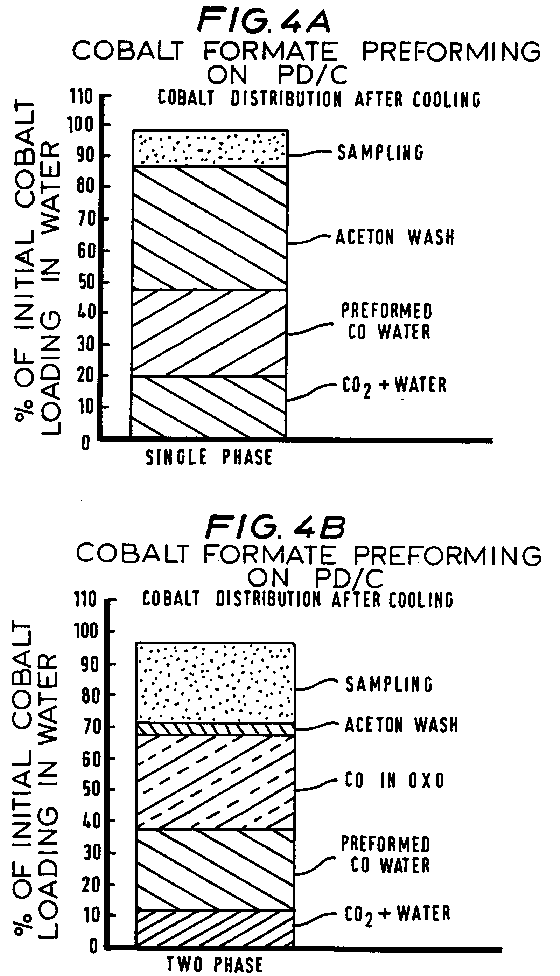

After cooling and reactor discharge, the catalyst was washed with acetone to recover the missing cobalt, and the amounts of cobalt in the aqueous and organic liquid phases, in the gas phase, and in the catalyst were determined, the cobalt distribution as determined being shown in Fig. 4A. Most of the cobalt retained in the catalyst during the reaction was in the form of water-insoluble crystalline precipitate.

(b) Two-liquid-phase system

1.01 kg of cobalt-containing aqueous phase of the same composition as that used in Example 2(a) were contacted with 85 g catalyst (0.8% palladium on carbon) in the presence of 0.85 kg isodecyl oxo product and syn gas ($H_2/CO$ = 1.3 : 1) at a temperature of 120°C and pressure of 300 atmospheres (about 3 x $10^7$ Pa). (the term "isodecyl" being used herein to refer to a C-10 product of the oxo reaction, which product, it will be appreciated, will normally consist of a mixture of C-10 isomeric aldehydes and alcohols.) The cobalt content of the aqueous phase was analysed at intervals over a period of 90 minutes. The measured $Co^{2+}$ and preformed cobalt contents of the aqueous phase and cobalt content of the organic phase are shown in Figure 3, in which the upper cross-hatched areas represent cobalt removed in sampling. After cooling and reactor discharge, the catalyst was washed with acetone to remove cobalt remaining therein, and the amounts of cobalt in the aqueous phase, the gas phase and in the catalyst were determined; the cobalt distribution is shown in Fig. 4B. In this case, it was found that only 3% of the cobalt had remained in the catalyst, a proportion of that cobalt being contained in liquid reactants retained in the catalyst.

From a comparison of Figures 2 and 3 and of Figures 4A and 4B it may be seen that the amount of cobalt lost is considerably greater in the one-liquid-phase system than in the two-liquid-phase system. The crystalline precipitate formed in the one-liquid-phase system would eventually lead to plugging of the reactor and deactivation of the catalyst, with substantial loss of cobalt, thus making commercial use of such a single-phase system impracticable.

## Claims

**1.** A method of recovery of cobalt values from a cobalt-containing aqueous phase, comprising contacting the aqueous phase, in the presence of syn gas, with a solid catalyst that is suitable for catalysing carbonylation of cobalt ions and with an organic solvent for hydrocobalt carbonyl and dicobalt octacarbonyl, the said solvent being substantially immiscible with water and being present in an amount of not less than 20% by volume, based on the combined volume of the solvent and the aqueous phase to be contacted with the solid catalyst, and recovering from the aqueous phase and organic liquid phase so obtained volatile cobalt carbonyl compounds.

**2.** A method as claimed in claim 1, wherein the aqueous phase and the organic liquid phase are subsequently treated in a stripping step in which the said phases are contacted with a stripping gas, volatile cobalt compounds being entrained in the stripping gas.

**3.** A method as claimed in claim 2, wherein the aqueous phase and the organic liquid phase are combined with oxo product from an oxo reactor before they are contacted with the stripping gas.

**4.** A method for recovery of cobalt values from the crude product of a cobalt-catalysed oxo reaction, the crude oxo product containing cobalt compounds in addition to an organic oxo product, the method comprising

treating the crude oxo product to remove volatile cobalt compounds;

contacting the crude oxo product with an aqueous phase of pH not greater than 7 to form an aqueous phase containing cobalt values; and

contacting the aqueous phase so obtained, in the presence of syn gas, with a solid catalyst that is suitable for catalysing carbonylation of cobalt ions and with an organic solvent for hydrocobalt carbonyl and dicobalt octacarbonyl, the said solvent being substantially immiscible with water, to obtain cobalt-containing aqueous and organic liquid phases and a cobalt-containing gas phase.

**5.** A method as claimed in claim 4, wherein at least one of the cobalt-containing aqueous and organic liquid phases and the cobalt-containing syn gas obtained is treated to remove volatile cobalt compounds therefrom.

**6.** A method as claimed in claim 4 or claim 5, wherein the crude oxo product is treated in a stripping step in which it is contacted with a stream of stripping gas in the presence of an aqueous phase of pH not greater than 7 to entrain volatile cobalt compounds in said stripping gas and to form an aqueous phase containing cobalt values.

**7.** A method as claimed in claim 6, wherein at least one of the cobalt-containing syn gas and the cobalt-containing aqueous and organic liquid phases is treated to remove volatile cobalt compounds therefrom, the said treatment comprising recycling one or more of that gas and those phases to the stripping step.

**8.** A method as claimed in claim 6 or claim 7, wherein the stripping gas is syn gas.

**9.** A method as claimed in any one of claims 6 to 8, wherein cobalt compounds that are removed from the oxo product by the stripping gas are recycled to the oxo reaction.

**10.** A method as claimed in claim 9, wherein, after contacting the crude product, the stripping gas is contacted with olefin feed for the oxo reactor, cobalt values in the said stripping gas becoming entrained in the olefin feed.

**11.** A method as claimed in any of claims 6 to 10, wherein, after contacting the solid catalyst, the cobalt-containing aqueous and organic liquid phases and the cobalt-containing syn gas are combined with crude oxo product and fed to the stripping step.

**12.** A method as claimed in any one of claims 4 to 11, wherein the aqueous phase is concentrated to a cobalt concentration of at least 0.5 wt % cobalt (calculated as metal) before it is contacted with the solid catalyst.

**13.** A method as claimed in claim 12, wherein at least a part of the aqueous effluent from the concentration step is recycled to form a part of the aqueous phase of pH not greater than 7 that is contacted with crude oxo product.

**14.** A method as claimed in claim 13, in which before the said aqueous effluent is contacted with the crude oxo product it is used as wash water in a washing step in which residual cobalt is removed from the oxo product after that has been treated to remove volatile compounds and contacted with an aqueous phase of pH not greater than 7.

**15.** A method as claimed in any one of claims 4 to 14, wherein the water used in the method is contained in a substantially closed loop, substantially no waste water being generated.

**16.** A method according to any one of claims 4 to 15, wherein the solvent for hydrocobalt carbonyl and dicobalt octacarbonyl is selected from decobalted crude oxo product, hydrogenated oxo product, oxo alcohols, light oxo fraction, heavy oxo fraction and olefins.

**17.** A method as claimed in any one of claims 4 to 16, wherein the solid catalyst is a noble metal catalyst.

**18.** A method as claimed in claim 17, wherein palladium, platinum or gold or a compound of one of those metals is used as the noble metal catalyst.

**19.** A method for recovery of cobalt values from the crude product of a cobalt catalysed oxo reaction, substantially as described herein with reference to Figure 1 of the drawings.

**20.** A method for recovery of cobalt values from the crude product of a cobalt catalysed oxo reaction, substantially as described in Example 1 herein.

21. An apparatus comprising

an oxo reactor;
a stripping means arranged for receiving crude oxo product from the reactor and comprising means for effecting contact between the said oxo product and syn gas in the presence of water or aqueous acid and means for separating the aqueous phase from the oxo product and syn gas;
a preformer arranged for receiving separated aqueous phase from the stripping means, the said preformer having means for containing a solid catalyst and having inlet means for the separated aqueous phase, for an organic liquid phase and for a gas, the preformer further comprising means for effecting contact between the liquid phases and the solid catalyst, and means for effecting contact between the aqueous phase and the organic liquid phase and the gas; and
recycling means for recycling to the stripping means effluent from the preformer comprising one or more of the organic liquid phase, the aqueous phase and the gas.

22. An oxo apparatus including a cobalt recovery system substantially as described with reference to Figure 1 of the drawings herein.

23. The use of a two-liquid-phase catalytic preformer for improving the efficiency of recovery of cobalt values.

## Patentansprüche

1. Verfahren zur Rückgewinnung von Kobaltwertstoffen aus einer Kobalt enthaltenden wäßrigen Phase, bei dem die wäßrige Phase in Gegenwart von Syngas mit einem Katalysator, der für die Katalysierung einer Carbonylierung von Kobaltionen geeignet ist, und mit einem organischen Lösungsmittel für Hydrokobaltcarbonyl und Dikobaltoctacarbonyl in Kontakt gebracht wird, wobei das Lösungsmittel im wesentlichen mit Wasser unmischbar ist und in einer Menge von nicht weniger als 20 Vol.-%, bezogen auf das kombinierte Volumen des Lösungsmittels und der wäßrigen Phase, die mit dem festen Katalysator in Kontakt gebracht werden soll, vorliegt, und aus der so erhaltenen wäßrigen Phase und der so erhaltenen organischen flüssigen Phase flüchtige Kobaltcarbonylverbindungen zurückgewonnen werden.

2. Verfahren nach Anspruch 1, bei dem die wäßrige Phase und die organische flüssige Phase anschließend in einem Strippschritt behandelt werden, bei dem diese Phasen mit einem Strippgas in Kontakt gebracht werden, wobei flüchtige Kobaltverbindungen in dem Strippgas mitgerissen werden.

3. Verfahren nach Anspruch 2, bei dem die wäßrige Phase und die organische flüssige Phase mit Oxoprodukt aus einem Oxoreaktor kombiniert werden, bevor sie mit dem Strippgas in Kontakt gebracht werden.

4. Verfahren zur Rückgewinnung von Kobaltwertstoffen aus dem Rohprodukt einer kobalt-katalysierten Oxoreaktion, wobei das rohe Oxoprodukt zusätzlich zu einem organischen Oxoprodukt Kobaltverbindungen enthält, bei dem

das rohe Oxoprodukt behandelt wird, um flüchtige Kobaltverbindungen zu entfernen,
das rohe Oxoprodukt mit einer wäßrigen Phase mit einem pH-Wert von nicht größer als 7 in Kontakt gebracht wird, um eine wäßrige Phase zu bilden, die Kobaltwertstoffe enthält, und
die so erhaltene wäßrige Phase in Gegenwart von Syngas mit einem festen Katalysator, der für die Katalysierung einer Carbonylierung von Kobaltionen geeignet ist, und einem organischen Lösungsmittel für Hydrokobaltcarbonyl und Dikobaltoctacarbonyl in Kontakt gebracht wird, wobei das Lösungsmittel mit Wasser im wesentlichen unmischbar ist, um Kobalt enthaltende wäßrige und organische flüssige Phasen und eine Kobalt enthaltende Gasphase zu erhalten.

5. Verfahren nach Anspruch 4, bei dem mindestens eine/eines von der erhaltenen Kobalt enthaltenden wäßrigen und organischen flüssigen Phasen und des erhaltenen Kobalt enthaltenen Syngases behandelt wird, um flüchtige Kobaltverbindungen daraus zu entfernen.

6. Verfahren nach Anspruch 4 oder Anspruch 5, bei dem das rohe Oxoprodukt in einem Strippschritt behandelt wird, bei dem es mit einem Strom von Strippgas in Gegenwart einer wäßrigen Phase mit einem pH-Wert von nicht größer als 7 in Kontakt gebracht wird, um flüchtige Kobaltverbindungen in dem Strippgas mitzureißen und um eine Kobaltwertstoffe enthaltende wäßrige Phase zu bilden.

7. Verfahren nach Anspruch 6, bei dem mindestens eines/eine von dem Kobalt enthaltenden Syngas und den Kobalt

enthaltenden wäßrigen und organischen flüssigen Phasen behandelt wird, um flüchtige Kobaltverbindungen daraus zu entfernen, wobei die Behandlung die Rückführung von einem oder mehreren von diesem Gas und diesen Phasen zu dem Strippschritt umfaßt.

**8.** Verfahren nach Anspruch 6 oder Anspruch 7, bei dem das Strippgas Syngas ist.

**9.** Verfahren nach einem der Ansprüche 6 bis 8, bei dem Kobaltverbindungen, die aus dem Oxoprodukt durch das Strippgas entfernt worden sind, zu der Oxoreaktion zurückgeführt werden.

**10.** Verfahren nach Anspruch 9, bei dem das Strippgas nach der Kontaktierung des Rohproduktes mit Olefineinsatzmaterial für den Oxoreaktor in Kontakt gebracht wird, wobei Kobaltwertstoffe in dem Strippgas in dem Olefineinsatzmaterial mitgerissen werden (eingeschlossen werden).

**11.** Verfahren nach einem der Ansprüche 6 bis 10, bei dem die Kobalt enthaltenden wäßrigen und organischen flüssigen Phasen und das Kobalt enthaltende Syngas nach der Kontaktierung des festen Katalysators mit rohem Oxoprodukt kombiniert und dem Strippschritt zugeführt werden.

**12.** Verfahren nach einem der Ansprüche 4 bis 11, bei dem die wäßrige Phase auf eine Kobaltkonzentration von mindestens 0,5 Gew.-% Kobalt (berechnet als Metall) aufkonzentriert wird, bevor sie mit dem festen Katalysator in Kontakt gebracht wird.

**13.** Verfahren nach Anspruch 12, bei dem mindestens ein Teil des aus dem Konzentrierungsschritt austretenden wäßrigen Materials zurückgeführt wird, um einen Teil der wäßrigen Phase mit einem pH-Wert von nicht größer als 7 zu bilden, die mit dem rohen Oxoprodukt in Kontakt gebracht wird.

**14.** Verfahren nach Anspruch 13, bei dem das wäßrige austretende Material, bevor es mit dem rohen Oxoprodukt in Kontakt gebracht wird, als Waschwasser in einem Waschschritt verwendet wird, bei dem restliches Kobalt aus dem Oxoprodukt entfernt wird, nachdem dieses behandelt worden ist, um flüchtige Verbindungen zu entfernen, und mit einer wäßrigen Phase mit einem pH-Wert von nicht größer als 7 in Kontakt gebracht worden ist.

**15.** Verfahren nach einem der Ansprüche 4 bis 14, bei dem das in diesem Verfahren verwendete Wasser in einem im wesentlichen geschlossenen Kreislauf enthalten ist, wobei im wesentlichen kein Abwasser erzeugt wird.

**16.** Verfahren nach einem der Ansprüche 4 bis 15, bei dem das Lösungsmittel für Hydrokobaltcarbonyl und Dikobaltoctacarbonyl ausgewählt ist aus von Kobalt befreitem rohem Oxoprodukt, hydriertem Oxoprodukt, Oxoalkoholen, leichten Oxofraktionen, schweren Oxofraktionen und Olefinen.

**17.** Verfahren nach einem der Ansprüche 4 bis 16, bei dem der feste Katalysator ein Edelmetallkatalysator ist.

**18.** Verfahren nach Anspruch 17, bei dem Palladium, Platin oder Gold oder eine Verbindung von einem dieser Metalle als Edelmetallkatalysator verwendet wird.

**19.** Verfahren zur Gewinnung von Kobaltwertstoffen aus dem Rohprodukt einer kobalt-katalysierten Oxoreaktion, das im wesentlichen wie unter Bezugnahme auf Figur 1 der Zeichnungen hierin beschrieben ist.

**20.** Verfahren zur Gewinnung von Kobaltwertstoffen aus dem Rohprodukt einer kobalt-katalysierten Oxoreaktion, das im wesentlichen wie in Beispiel 1 hierin beschrieben ist.

**21.** Vorrichtung, die

einen Oxoreaktor,
Strippmittel, die zur Aufnahme von rohem Oxoprodukt aus dem Reaktor angeordnet sind und Mittel zur Bewirkung eines Kontaktes zwischen dem Oxoprodukt und Syngas in Gegenwart von Wasser oder wäßriger Säure und Mittel zur Abtrennung der wäßrigen Phase von dem Oxoprodukt und dem Syngas umfassen,
einen Vorformer, der zur Aufnahme von abgetrennter wäßriger Phase aus den Strippmitteln angeordnet ist, wobei der Vorformer Mittel zum Enthalten eines festen Katalysators und Einlaßmittel für die abgetrennte wäßrige Phase, für eine organische flüssige Phase und für ein Gas aufweist, wobei der Vorformer ferner Mittel zum Bewirken eines Kontaktes zwischen den flüssigen Phasen und dem festen Katalysator und Mittel zur Bewirkung eines Kontaktes zwischen der wäßrigen Phase und der organischen flüssigen Phase und dem Gas

umfaßt, und

Rückführungsmittel zum Rückführen von aus dem Vorformer austretenden Material, das eine oder mehrere der organischen flüssigen Phase, der wäßrigen Phase und des Gases umfaßt, zu den Strippmitteln umfaßt.

**22.** Oxovorrichtung, die ein Kobaltrückgewinnungssystem umfaßt, das im wesentlichen wie unter Bezugnahme auf Figur 1 der Zeichnungen hierin beschrieben ist.

**23.** Verwendung eines katalytischen Zwei-Flüssigphasen-Vorformers zur Verbesserung der Effizienz der Rückgewinnung von Kobaltwertstoffen.

## Revendications

**1.** Procédé pour récupérer la teneur en cobalt d'une phase aqueuse contenant du cobalt, comprenant la mise en contact de la phase aqueuse, en présence de gaz de synthèse, avec un catalyseur solide qui convient pour catalyser la carbonylation des ions cobalt et avec un solvant organique pour l'hydrocobalt-carbonyle et le dicobalt-octacarbonyle, le solvant en question étant sensiblement non miscible à l'eau et étant présent en une quantité non inférieure à 20 % en volume, sur la base du volume total du solvant et de la phase aqueuse devant entrer en contact avec le catalyseur solide, et la récupération des composés formés de cobalt-carbonyles volatils ainsi obtenus de la phase aqueuse et de la phase liquide organique.

**2.** Procédé suivant la revendication 1, dans lequel la phase aqueuse et la phase liquide organique sont ensuite traitées dans une étape d'entraînement dans laquelle les phases en question sont mises en contact avec un gaz d'entraînement, les composés de cobalt volatils étant entraînés dans le gaz d'entraînement.

**3.** Procédé suivant la revendication 2, dans lequel la phase aqueuse et la phase liquide organique sont mélangées à un produit oxo provenant d'un réacteur oxo avant d'être mises en contact avec le gaz d'entraînement.

**4.** Procédé pour récupérer la teneur en cobalt du produit brut d'une réaction oxo catalysée au cobalt, le produit oxo brut contenant des composés de cobalt en plus d'un produit oxo organique, ce procédé comprenant

le traitement du produit oxo brut pour en séparer les composés de cobalt volatils ;

la mise en contact du produit oxo brut avec une phase aqueuse de pH non supérieur à 7 pour former une phase aqueuse contenant du cobalt ; et

à faire entrer la phase aqueuse ainsi obtenue, en présence de gaz de synthèse, en contact avec un catalyseur solide qui convient pour catalyser la carbonylation des ions cobalt et avec un solvant organique pour l'hydrocobalt-carbonyle et le dicobalt-octacarbonyle, ce solvant étant sensiblement non miscible à l'eau, afin d'obtenir des phases liquides aqueuse et organique contenant du cobalt et une phase gazeuse contenant du cobalt.

**5.** Procédé suivant la revendication 4, dans lequel l'un au moins des phases liquides aqueuse et organique contenant du cobalt et du gaz de synthèse contenant du cobalt qui sont obtenus est traité pour en éliminer les composés de cobalt volatils.

**6.** Procédé suivant la revendication 4 ou la revendication 5, dans lequel le produit oxo brut est traité dans une étape d'entraînement dans laquelle il est mis en contact avec un courant de gaz d'entraînement en présence d'une phase aqueuse de pH non supérieur à 7 afin d'entraîner les composés de cobalt volatils dans ce gaz d'entraînement pour former une phase aqueuse contenant du cobalt.

**7.** Procédé suivant la revendication 6, dans lequel l'un au moins du gaz de synthèse contenant du cobalt et des phases liquides aqueuse et organique contenant du cobalt est traité afin d'en éliminer les composés de cobalt volatils, ce traitement comprenant le recyclage d'un ou plusieurs de ce gaz et de ces phases à l'étape d'entraînement.

**8.** Procédé suivant la revendication 6 ou la revendication 7, dans lequel le gaz d'entraînement est du gaz de synthèse.

**9.** Procédé suivant l'une quelconque des revendications 6 à 8, dans lequel les composés de cobalt qui sont éliminés du produit oxo par le gaz d'entraînement sont recyclés dans la réaction oxo.

**10.** Procédé suivant la revendication 9, dans lequel, après contact avec le produit brut, le gaz d'entraînement est mis en contact avec une phase oléfinique destinée au réacteur oxo, le cobalt présent dans ce gaz d'entraînement étant entraîné dans la charge oléfinique.

**11.** Procédé suivant l'une quelconque des revendications 6 à 10, dans lequel, après contact avec le catalyseur solide, les phases liquides aqueuse et organique contenant du cobalt et le gaz de synthèse contenant du cobalt sont réunies avec le produit oxo brut et le mélange est chargé dans l'étape d'entraînement.

**12.** Procédé suivant l'une quelconque des revendications 4 à 11, dans lequel la phase aqueuse est concentrée à une concentration en cobalt d'au moins 0,5 % en poids de cobalt (exprimée en métal) avant sa mise en contact avec le catalyseur solide.

**13.** Procédé suivant la revendication 12, dans lequel une partie au moins de l'effluent aqueux de l'étape de concentration est recyclée pour former une partie de la phase aqueuse de pH non supérieur à 7 qui est mise en contact avec le produit oxo brut.

**14.** Procédé suivant la revendication 13, dans lequel, avant la mise en contact de l'effluent aqueux avec le produit oxo brut, il est utilisé comme eau de lavage dans une étape de lavage dans laquelle le cobalt résiduel est éliminé du produit oxo après qu'il a été traité pour éliminer les composants volatils et mis en contact avec une phase aqueuse de pH non supérieur à 7.

**15.** Procédé suivant l'une quelconque des revendications 4 à 14, dans lequel l'eau utilisée dans ce procédé est contenue dans un circuit sensiblement fermé, sensiblement sans génération d'eau résiduaire.

**16.** Procédé suivant l'une quelconque des revendications 4 à 15, dans lequel le solvant pour l'hydrocobalt-carbonyle et le dicobalt-octacarbonyle est choisi entre le produit oxo brut débarrassé du cobalt, le produit oxo hydrogéné, des oxo-alcools, une fraction oxo légère, une fraction oxo lourde et des oléfines.

**17.** Procédé suivant l'une quelconque des revendications 4 à 16, dans lequel le catalyseur solide est un catalyseur à base de métal noble.

**18.** Procédé suivant la revendication 17, dans lequel du palladium, du platine ou de l'or ou un composé de l'un de ces métaux est utilisé comme catalyseur à base de métal noble.

**19.** Procédé pour récupérer la teneur en cobalt du produit brut d'une réaction oxo catalysée au cobalt, sensiblement tel que décrit en regard de la figure 1 des dessins.

**20.** Procédé pour récupérer la teneur en cobalt du produit brut d'une réaction oxo catalysée au cobalt, sensiblement tel que décrit en regard de la figure 1.

**21.** Appareil comprenant

un réacteur oxo ;
un moyen d'entraînement conçu pour recevoir le produit oxo brut du réacteur et comprenant un moyen pour établir le contact entre ce produit oxo et du gaz de synthèse en présence d'eau ou d'un acide aqueux et un moyen pour séparer la phase aqueuse du produit oxo et du gaz de synthèse ;
un dispositif de préformage conçu pour recevoir la phase aqueuse séparée du moyen d'entraînement, ce dispositif de préformage étant équipé de moyens permettant de contenir un catalyseur solide et d'un moyen d'admission de la phase aqueuse séparée, d'une phase liquide organique et d'un gaz, le dispositif de préformage comprenant en outre un moyen permettant d'établir un contact entre les phases liquides et le catalyseur solide, et un moyen établissant le contact entre les phases liquides aqueuse et organique et le gaz ; et
un moyen servant à recycler au moyen d'entraînement l'effluent du dispositif de préformage comprenant un ou plusieurs de la phase liquide organique, de la phase aqueuse et du gaz.

**22.** Appareil oxo comprenant un système de récupération du cobalt sensiblement tel que décrit en regard de la figure 1 des dessins annexés.

**23.** Utilisation d'un dispositif de préformage catalytique à deux phases liquides pour améliorer le rendement de récupération de la teneur en cobalt.

FIG.1

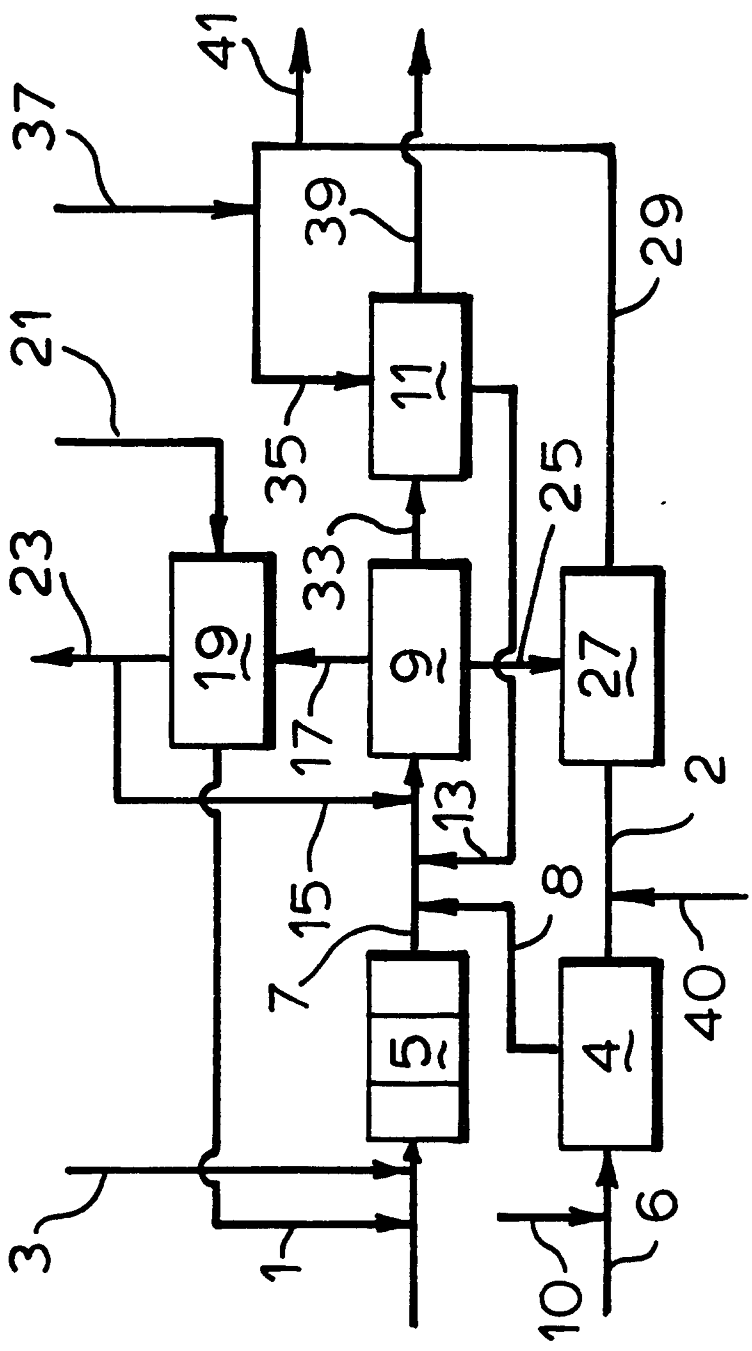

FIG. 2

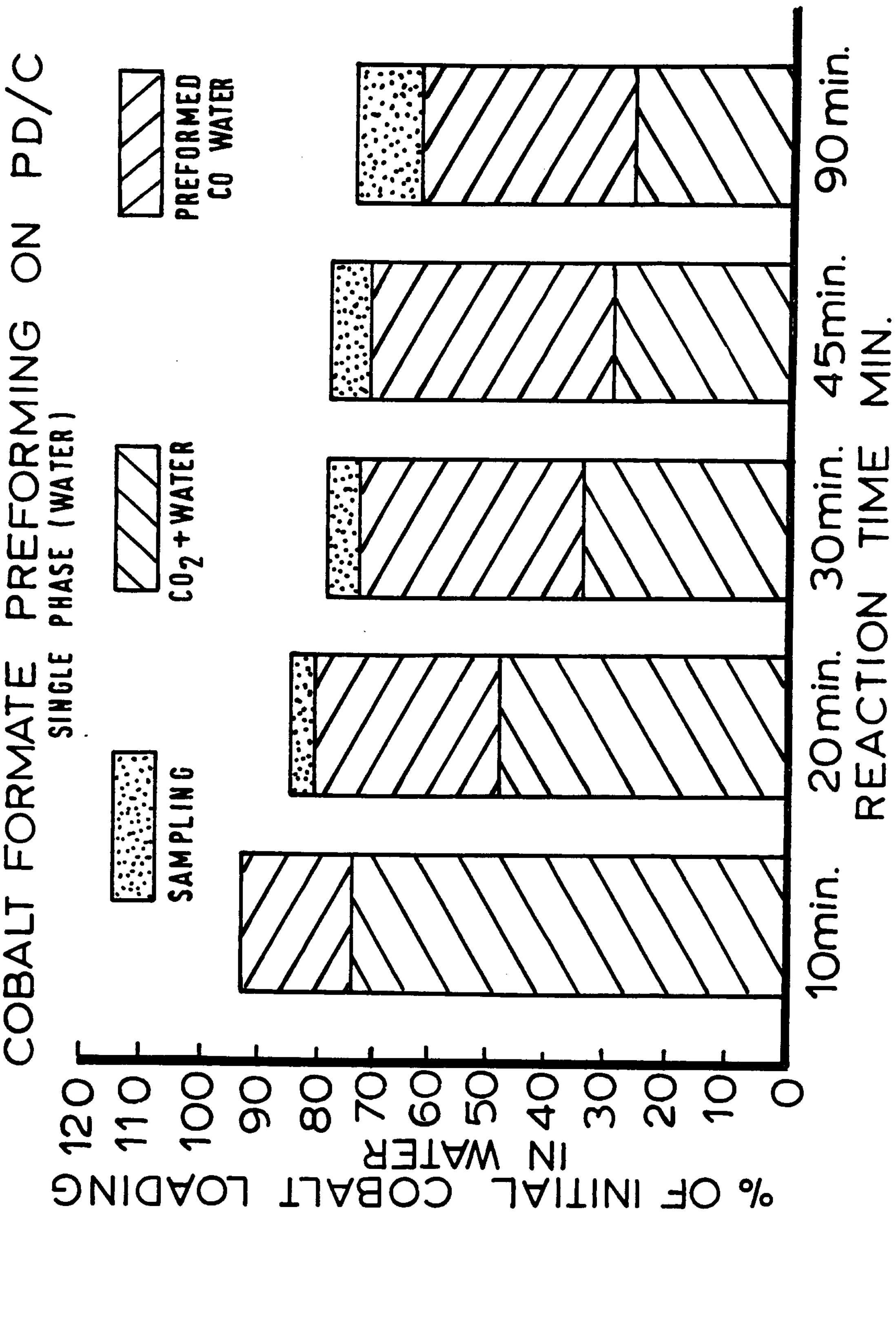
COBALT FORMATE PREFORMING ON PD/C
SINGLE PHASE (WATER)
SAMPLING
$CO_2$ + WATER
PREFORMED CO WATER
% OF INITIAL COBALT LOADING IN WATER
120
110
100
90
80
70
60
50
40
30
20
10
0
10min.
20min.
30min.
45min.
90min.
REACTION TIME MIN.

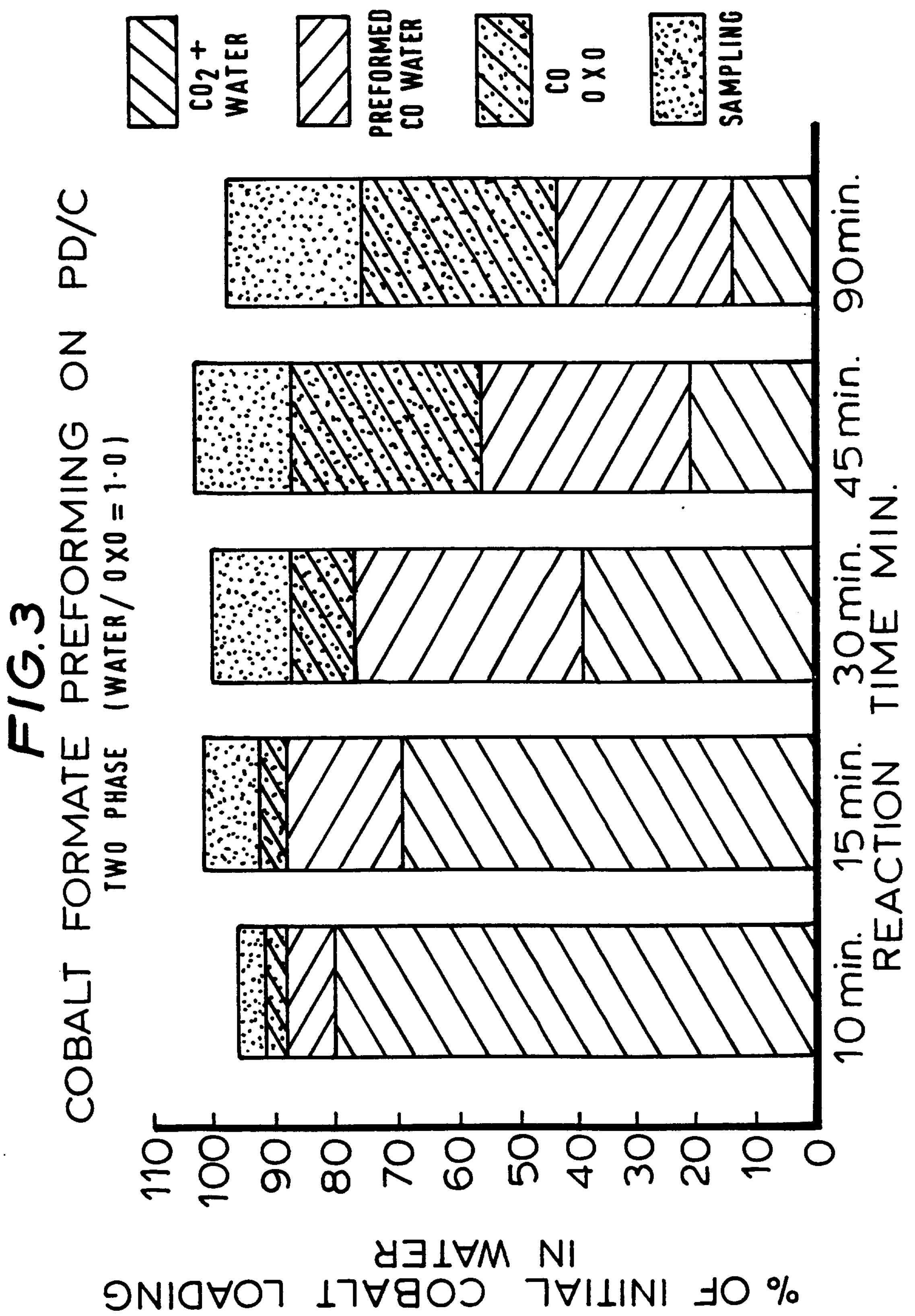
COBALT FORMATE PREFORMING ON PD/C
TWO PHASE (WATER/OXO = 1·0)
% OF INITIAL COBALT LOADING IN WATER
0
10
20
30
40
50
60
70
80
90
100
110
10 min.
15 min.
30 min.
45 min.
90 min.
REACTION TIME MIN.
$CO_2$ + WATER
PREFORMED CO WATER
CO OXO
SAMPLING

FIG.3

*FIG. 4A*

COBALT FORMATE PREFORMING ON PD/C

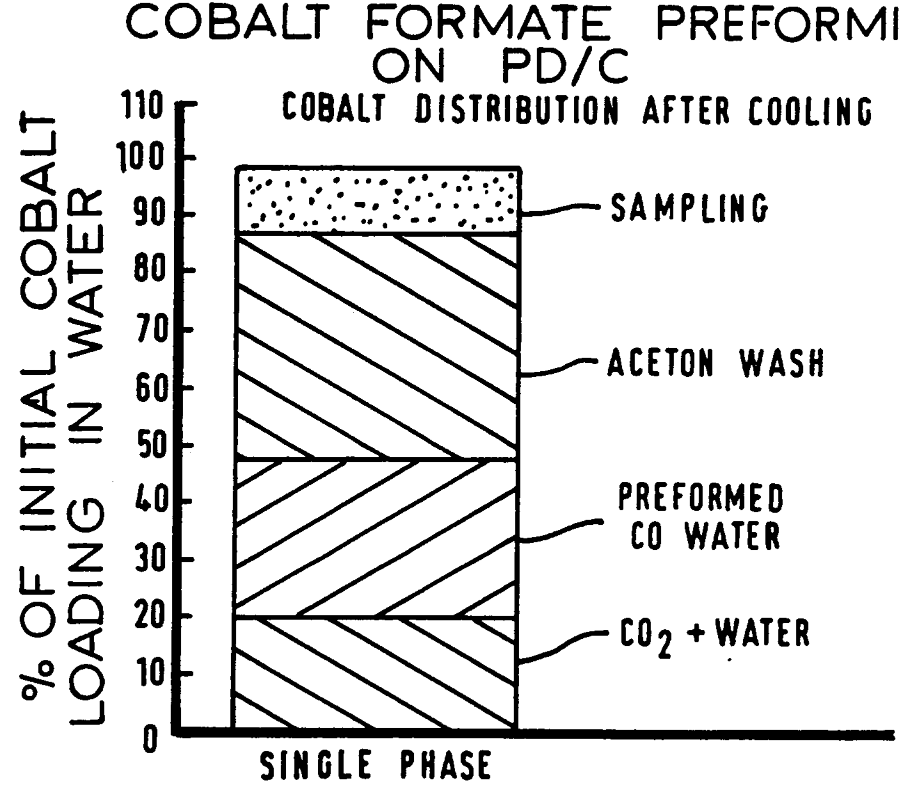


*FIG. 4B*

COBALT FORMATE PREFORMING ON PD/C